# EUROPEAN PATENT APPLICATION

(11) **EP 4 564 006 A1**
(43) Date of publication of application: **04.06.2025**
(21) Application number: 22952487.1
(22) Date of filing: 29.07.2022
(51) Int. Cl.: G01N 33/58, G01N 33/53, B01L 3/00

(54) **CELL IDENTIFICATION METHOD**

(71) Applicant: Tseng, Fan-Gang, Hsinchu Taiwan 30013 (TW)
(72) Inventor: WU, Jen-Kuei, Hsinchu City, Taiwan 30013 (TW); TSENG, Fan-Gang, Hsinchu City, Taiwan 30013 (TW)
(74) Representative: Becker, Eberhard
(86) International application number: PCT/CN2022/109035
(87) International publication number: WO 2024/021040

(57) **Abstract**

Provided are a cell identification method for identifying target cells by combining the results of fluorescence intensity analysis, fluorescence image analysis, and white light image analysis.

## Description

### Field of the Invention

The invention relates to a cell identification method, and more particularly, to an identification method capable of identifying rare cells with a high accurate identification rate.

### Description of Related Art

Cells with a count less than 1000 in a milliliter of blood sample may be classified as rare cells. Rare cells such as circulating tumor cells (CTCs) and fetal nucleated red blood cells (FnRBCs) may be used in fields such as medicine, detection, and analysis, such as auxiliary cancer prognosis analysis, prenatal detection, or virus infection detection and analysis.

For the sorting and identification of rare cells in blood, clinically, blood smears are used to identify whether there are target cells under the microscope via chemical staining. However, since the number ratio of target cells to background cells (such as red blood cells or white blood cells) is too low (about 1:10⁷ to 10⁹) when sorting and identifying target cells according to this method, and at the same time, when using specific antibodies to mark target cells to identify target cells, the binding rate and accuracy of target cells and antibodies do not yet meet the minimum standard (99.5%) for clinical identification. Therefore, it is necessary to establish a more accurate identification logic or analysis method.

### SUMMARY OF THE INVENTION

The invention provides a cell identification method, and more specifically, an identification method capable of identifying rare cells with a high accurate identification rate.

A cell identification method of the invention includes the following steps. An analysis sample is provided, wherein the analysis sample contains target cells, non-target cells, and fluorescent markers, and the fluorescent markers include a first fluorescent marker for marking nuclei, a second fluorescent marker for marking the target cells, and a third fluorescent marker for marking the target cells or the non-target cells. A fluorescence scan is performed on the analysis sample respectively with a first fluorescence waveband corresponding to the first fluorescent marker, a second fluorescence waveband corresponding to the second fluorescent marker, and a third fluorescence waveband corresponding to the third fluorescent marker to obtain a first fluorescence image photo, a second fluorescence image photo, and a third fluorescence image photo of the analysis sample in the first fluorescence waveband, the second fluorescence waveband, and the third fluorescence waveband. A fluorescence signal brightness of the first fluorescence image photo, the second fluorescence image photo, and the third fluorescence image photo is measured, and a fluorescence signal with a fluorescence signal brightness falling within a preset range is marked as a valid fluorescence signal.

According to an embodiment of the invention, cells having valid fluorescence signals in both the first fluorescence image photo and the second fluorescence image photo are marked as first preliminary target cells. When the third fluorescent marker is used to mark the target cells, selection is made from the first preliminary target cells, and cells having valid fluorescence signals in the third fluorescence image photo are screened and marked as second preliminary target cells. Alternatively, when the third fluorescent marker is used to mark the non-target cells, selection is made from the first preliminary target cells, and the cells that do not have valid fluorescence signals in the third fluorescence image photo are screened and marked as the second preliminary target cells. A white light scan is performed on the analysis sample with a white light to obtain a white light image photo of the analysis sample, selection is made from the second preliminary target cells, and the cells having a specific cell phenotype and shape are selected for further screening and identification as the target cells.

In an embodiment of the invention, the target cells are circulating tumor cells, the first fluorescent marker is Hoechst 33342, the second fluorescent marker is an anti-EpCAM antibody with fluorescein isothiocyanate (FITC), and the third fluorescent marker is an anti-CD45 antibody with phycoerythrin (PE).

In an embodiment of the invention, the target cells are fetal nucleated red blood cells, the first fluorescent marker is 4',6-diamidino-2-phenylindole (DAPI), the second fluorescent marker is an anti-CD147 antibody with FITC, and the third fluorescent marker is an anti-CD71 antibody with PE.

In an embodiment of the invention, the target cells are cells that undergo epithelial-mesenchymal transition (EMT) (also known as EMT cells for short), the first fluorescent marker is Hoechst 33342 or DAPI, the second fluorescent marker is the anti-EpCAM antibody with FITC, and the third fluorescent marker is an anti-vimentin antibody with PE.

In an embodiment of the invention, the first fluorescent marker is Hoechst 33342, and a brightness range of a valid fluorescence signal of the first fluorescent marker is 5 to 40.

In an embodiment of the invention, the second fluorescent marker is the anti-EpCAM antibody with FITC, and a brightness range of a valid fluorescence signal of the second fluorescent marker is 70 to 120.

In an embodiment of the invention, the third fluorescent marker is the anti-CD45 antibody with PE, and a brightness range of the valid fluorescence signal of the third fluorescent marker is 3 to 45.

In an embodiment of the invention, the method step of performing the white light scan on the analysis sample to obtain the white light image photo of the analysis sample and further identifying and screening from the second preliminary target cells to identify the cells having the specific cell phenotype and shape as the target cells includes: calculating, according to the obtained white light image photo, a perimeter and an area of the marked second preliminary target cells to determine whether a shape and a size of each cell of the marked second preliminary target cells conform to a standard shape of the target cells, to further determine and identify whether the marked second preliminary cells are the target cells.

In an embodiment of the invention, the standard shape of the target cells includes a circle or a micro-ellipse with an eccentricity less than 0.8.

In an embodiment of the invention, the step of identifying the second preliminary target cells having the specific cell phenotype and shape as the target cells includes: cross-comparing the obtained white light image photo with the second fluorescence image photo, and confirming whether a relative position error between a cell contour of the second preliminary target cells marked in the white light image and a cell contour located by the valid fluorescence signals in the second fluorescence image is less than or equal to a specific ratio of a diameter of the target cells.

In an embodiment of the invention, the specific ratio is 5% of the diameter of the target cells.

### BRIEF DESCRIPTION OF THE DRAWINGS

FIG. 1 is a schematic diagram of a main flowchart of a cell identification method according to an embodiment of the invention.
FIG. 2 is a schematic diagram of a detailed flowchart of a cell identification method according to an embodiment of the invention.
FIG. 3 is a schematic diagram of a detailed flowchart of a cell identification method according to an embodiment of the invention.
FIG. 4 is a schematic diagram of a detailed flowchart of a cell identification method according to an embodiment of the invention.
FIG. 5 is a fluorescence image photo according to an embodiment of the invention.
FIG. 6 is a flowchart of interpretation logic of the fluorescence image analysis of a cell identification method according to an embodiment of the invention.
FIG. 7 is an enlarged view of a specific position of a fluorescence image photo of different fluorescence wavebands according to an embodiment of the invention.
FIG. 8 is an enlarged view of a specific position of a fluorescence image photo of different fluorescence wavebands according to an embodiment of the invention.
FIG. 9 is a white light image photo according to an embodiment of the invention.

### DESCRIPTION OF THE EMBODIMENTS

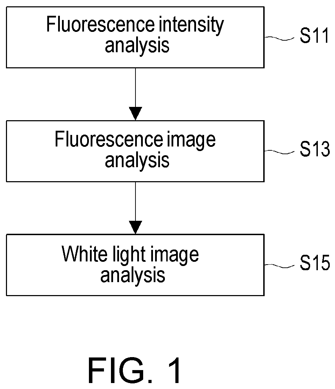

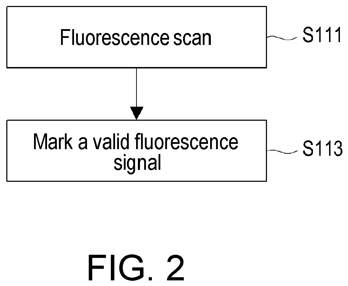

FIG. 1 is a schematic diagram of a main flowchart of a cell identification method according to an embodiment of the invention. FIG. 2 to FIG. 4 are schematic diagrams of a detailed flowchart of a cell identification method according to an embodiment of the invention.

Referring to FIG. 1, the cell identification method according to an embodiment of the invention mainly includes three main processes S11, S13, and S15. The process S11 is to analyze the fluorescence intensity of an analysis sample containing target cells, non-target cells, and fluorescent markers, and determine whether it is valid fluorescence according to the brightness range of the fluorescence. The process S13 is to interpret the fluorescence image analysis of preliminary target cells according to whether the valid fluorescence signals are overlapped. The process S15 is white light image analysis for evaluating the cell phenotype and shape of the preliminary target cells according to the white light image photo of the preliminary target cells.

The detailed flow of the fluorescence intensity analysis is described in detail with reference to FIG. 2. First, fluorescence scan is performed on an analysis sample containing target cells, non-target cells, and fluorescent markers with different fluorescence excitation wavebands to obtain a fluorescence image photo of the fluorescent markers (process S111). Next, the brightness of fluorescence signals in the fluorescence image photo is measured, and fluorescence signals with a fluorescence signal brightness within a predetermined range are marked as valid fluorescence signals (process S113). The brightness range of the valid fluorescence signals of various fluorescent markers was obtained based on the linear brightness range provided by the calibration of the linear concentration curve of standard epithelial cancer cells.

In an embodiment of the invention, the fluorescent markers may include a first fluorescent marker corresponding to a first fluorescence waveband, a second fluorescent marker corresponding to a second fluorescence waveband, and a third fluorescent marker corresponding to a third fluorescence waveband, wherein the first fluorescent marker may be used to mark specific members of cells such as nuclei, the second fluorescent marker may be used to mark target cells, and the third fluorescent marker may be used to mark target cells or mark non-target cells.

In some embodiments of the invention, fluorescent markers include fluorescent markers marking target cells, fluorescent markers marking nuclei, and fluorescent markers marking non-target cells to facilitate screening.

In some embodiments of the invention, fluorescent markers include two different fluorescent markers marking target cells and a fluorescent marker marking nuclei for screening.

For example, the first fluorescent marker may be, for example, 4',6-diamidino-2-phenylindole (DAPI) or the blue fluorescent dye Hoechst 33342 bound to the nuclei to generate blue fluorescence corresponding to the first waveband. The second fluorescent marker may generate green fluorescence corresponding to the second waveband due to the specific fluorescent substance it carries, such as fluorescein isothiocyanate (FITC). The third fluorescent marker may generate orange-yellow fluorescence corresponding to the third waveband due to the specific fluorescent substance it carries, such as phycoerythrin (PE).

For example, when the target cells are circulating tumor cells (CTCs), the first fluorescent marker may be, for example, the blue fluorescent dye Hoechst 33342 or DAPI bound to the nuclei, the second fluorescent marker may be, for example, the anti-EpCAM antibody with a specific fluorescent substance FITC marking circulating tumor cells, and the third fluorescent marker may be, for example, the anti-CD45 antibody with a specific fluorescence substance PE bound to non-target cells such as white blood cells or red blood cells.

For example, when the target cells are fetal nucleated red blood cells (FnRBCs), two different fluorescent markers may be used to identify the target cells. The first fluorescent marker may be, for example, the blue fluorescent dye Hoechst 33342 or DAPI bound to the nuclei, the second fluorescent marker may be, for example, an anti-CD147 antibody with a specific fluorescent substance FITC marking target cells, and the third fluorescent marker may be, for example, the anti-CD71 antibody with a fluorescent substance PE marking target cells.

For example, when the target cells are circulating tumor cell clusters (CTMs), the first fluorescent marker may be, for example, the blue fluorescent dye Hoechst 33342 bound to the nuclei, the second fluorescent marker may be, for example, the anti-EpCAM antibody with a specific fluorescent substance FITC marking circulating tumor cells, and the third fluorescent marker may be, for example, an anti-CD8 antibody with a specific fluorescent substance PE marking cytotoxic T cells.

For example, when the target cells are cells undergoing epithelial-mesenchymal transition (EMT) (also known as EMT cells for short), the first fluorescent marker may be, for example, a blue fluorescent dye Hoechst 33342 or DAPI bound to the nuclei, the second fluorescent marker may be, for example, the anti-EpCAM antibody with a specific fluorescence substance FITC marking EMT cells, and the third fluorescent marker may be, for example, an anti-vimentin antibody with a specific fluorescent substance PE marking EMT cells. In some embodiments of the invention, the fluorescence scan (process S111) includes fluorescence scan of the analysis sample in the first fluorescence waveband, the second fluorescence waveband, and the third fluorescence waveband to obtain a first fluorescence image photo, a second fluorescence image photo, and a third fluorescence image photo of the analysis sample in the first fluorescence waveband, the second fluorescence waveband, and the third fluorescence waveband. Marking the valid fluorescence signals (process S113) includes measuring a fluorescence signal brightness of the first fluorescence image photo, the second fluorescence image photo, and the third fluorescence image photo, and marking fluorescence signals with a fluorescence signal brightness falling within a preset range as valid fluorescence signals.

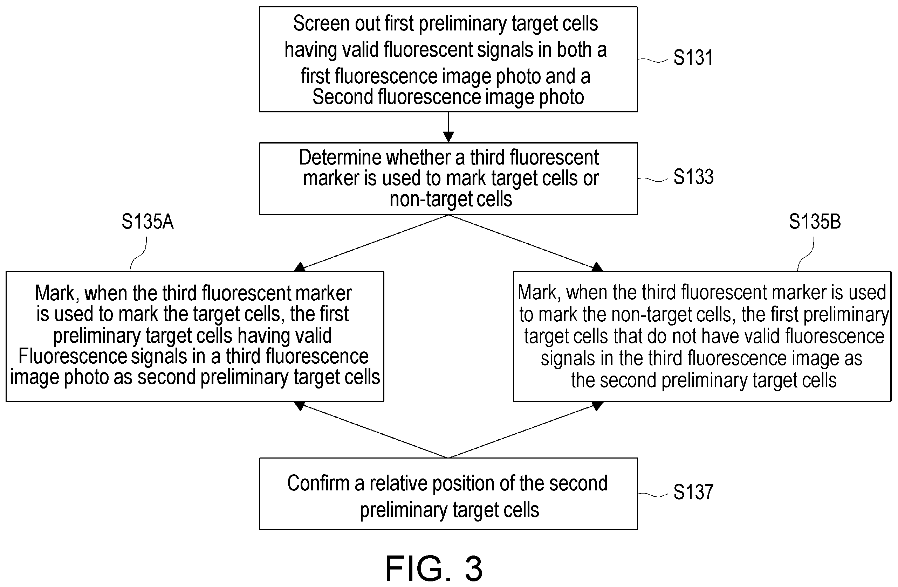

The detailed flow of the fluorescence image analysis is described in detail with reference to FIG. 3. First, the first fluorescence image photo, the second fluorescence image photo, and the third fluorescence image photo are overlapped to screen out the first preliminary target cells having valid fluorescence signals in both the first fluorescence image photo and the second fluorescence image photo (process S131). Next, whether the third fluorescent marker is used to mark target cells or non-target cells is determined (process S133). When the third fluorescent marker is used to mark the target cells, the first preliminary target cells having valid fluorescence signals in the third fluorescence image photo are marked as second preliminary target cells (process S135A), or, when the third fluorescent marker is used to mark the non-target cells, the first preliminary target cells that do not have valid fluorescence signals in the third fluorescence image photo are marked as the second preliminary target cells (process S135B). Lastly, the relative position (i.e., localization) of the second preliminary target cells is determined (process S137). Although FIG. 3 shows that the first preliminary cells are firstly screened according to the first fluorescence image photo showing the valid fluorescence signals of the target cell nuclei and the second fluorescence image photo showing the valid fluorescence signals of the target cells, and then the second preliminary cells are screened out according to the third fluorescence image photo showing the valid fluorescence signals marking the target cells or the non-target cells, the invention is not limited thereto.

According to an embodiment of the invention, the first preliminary cells may be screened out according to the second fluorescence image photo showing the valid fluorescence signals of the target cells and the third fluorescence image photo not showing the valid fluorescent signals of the non-target cells, then, the second preliminary cells are screened out according to the first fluorescence image photo showing the valid fluorescence signals of the marked nuclei.

According to an embodiment of the invention, the first preliminary cells may be screened out according to the second fluorescence image photo showing the valid fluorescence signals of the target cells and the third fluorescence image photo showing the valid fluorescent signals of the target cells, then, the second preliminary cells are screened out according to the first fluorescence image photo showing the valid fluorescence signals of the marked nuclei.

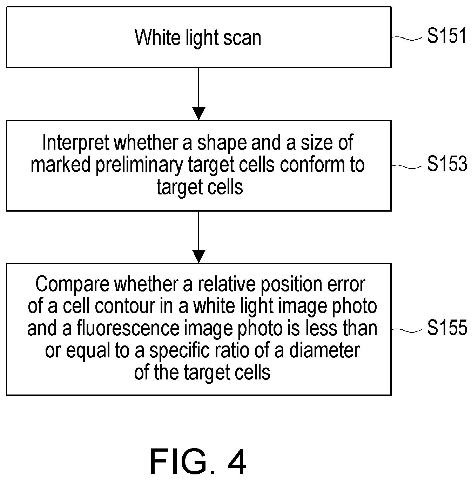

The detailed flow of the white light image analysis is described in detail with reference to FIG. 4. First, white light scan is performed on an analysis sample with a white light source (bright field) to obtain a white light image photo of the analysis sample (process S151). Next, according to the white light image photo of the analysis sample, the perimeter and area of the marked second preliminary target cells are calculated to determine whether the shape and size of the marked second preliminary target cells conform to the standard shape of the target cells (for example, a circle or an ellipse) (process S153). Next, the obtained white light image photo is cross-compared with the second fluorescence image having the valid fluorescence signals of the target cells to confirm whether the relative position error of the cell contour of the second preliminary target cells marked in the white light image photo and the cell contour located according to the valid fluorescence signals of the marked target cells in the second fluorescence image photo is less than or equal to a specific ratio of the diameter of the target cells (process S155). For example, when the shape of the cells is interpreted as a circle or a micro-ellipse conforming to the standard shape of the target cells, and the relative position error of the cell contour between the white light image and the fluorescence image is less than or equal to 5% of the diameter size of the target cells, the cells are identified as the target cells. Although FIG. 4 shows that the process S153 is executed first and then the process S155 is executed, the invention is not limited thereto. According to an embodiment of the invention, after comparing the relative positions of the cell contours in the white light image and the fluorescence image, whether the shape and size of the marked preliminarily target cells conform to the standard shape of the target cells may be interpreted.

Specific examples are provided below to describe the cell identification method according to an embodiment of the invention in detail.

First, an analysis sample is prepared.

For example, a physiological sample (e.g., saliva, secretion, blood sample, etc.) is obtained from a test subject to provide as a test sample. For example, the detection object is human or mammal, and the blood sample is, for example, whole blood, but not limited thereto.

Next, the provided blood sample is centrifuged to obtain a cell mixture, and the separated cell mixture contains the target cells and non-target cells that are predetermined to be identified in an embodiment of the present application. The cells in the cell mixture are mainly, for example, non-adherent cells (i.e., suspension cells), and, for example, include monocytes, circulating tumor cells (CTCs), fetal nucleated red blood cells (FnRBCs), cells undergoing epithelial-mesenchymal transition (EMT), or a combination thereof. In particular, according to certain embodiments, the target cells may include, for example, circulating tumor cells, and the non-target cells may include, for example, white blood cell count cells (WBC), red blood cells (RBC), and/or platelets, etc., but not limited thereto.

Specifically, for example, Ficoll-Paque^{™} cell/monosphere separation solution (solution prepared with Ficoll and sodium diatrizoate in proportion to a density of 1.077 g/ml) may be used to stratify blood components according to a density gradient, and the operation steps are roughly as follows: first, Lymphoprep^{™} is added dropwise below the filter membrane of the Leucosep centrifuge tube. Next, the blood sample is poured slowly along the wall of the centrifuge tube and centrifuged at 800×g (RCF, relative centrifugal force) for 15 minutes.

Next, the separated cell mixture is pipetted into a microcentrifuge tube, and a fluorescent marker is added to the cell mixture to bind the fluorescent marker to the target cells in the cell mixture. That is, the target cells are fluorescently stained to obtain an analysis sample. Specifically, the liquid (including the cell mixture and plasma) above the filter membrane in the centrifuge tube is transferred to another new centrifuge tube and centrifuged at 300×g for 10 minutes to allow the cells in the cell mixture to settle to the bottom of the centrifuge tube. Next, the supernatant is removed, and after 1 milliliter (ml) of phosphate buffered saline (PBS) is added to resuspend the cells in the cell mixture, a portion of the suspension (about 5 micro liters (µl)) is removed for cell counting, and the remaining suspension is centrifuged at 400×g for 6 minutes to allow the cells in the cell mixture to settle to the bottom of the centrifuge tube. Then, two-stage fluorescent staining is performed. The first-stage fluorescent staining is, for example, staining the cell surface antigens, and the second-stage fluorescent staining is, for example, staining the cell nuclei, and the exemplary steps are roughly as follows: first, the supernatant is removed, and after 100 *µ*l of PBS is added to resuspend the cells in the cell mixture, the first-stage fluorescent marker is added and protected from light for 30 minutes, to allow the first-stage fluorescent marker to be bound to specific cells in the cell mixture. Next, 1 ml of PBS is added, and the supernatant is removed after centrifugation at 400×g for 6 minutes to remove the first-stage fluorescent markers not bound to cell surface antigens. Then, after the cells in the cell mixture are resuspended by adding 100 *µ*l of PBS, the second-stage fluorescent marker is added and protected from light for 10 minutes to allow the second-phase fluorescent marker to be bound to the nuclei of all cells in the cell mixture. Next, 1 ml of PBS is added, centrifuged at 400×g for 6 minutes and the supernatant is removed to remove the second-stage fluorescent marker not bound to cells.

For example, when the target cells are circulating tumor cells, fluorescent markers in the first stage may include the anti-EpCAM antibody with a specific fluorescence substance, wherein the specific fluorescence substance is, for example, fluorescein isothiocyanate (FITC), which has a fluorescence waveband of excitation wavelength Ex: 482 ± 25 nm/emission wavelength Em: 531 ± 40 nm, which emits green fluorescence after being excited. The second-stage fluorescent marker may include the dye Hoechst 33342. Hoechst 33342 has a fluorescence waveband of excitation wavelength Ex: 357 ± 44 nm/emission wavelength Em: 475 ± 28 nm, which emits blue fluorescence after being excited. The anti-EpCAM antibody is bound to the surface antigen epithelial cell adhesion molecule (EpCAM) of circulating tumor cells, and Hoechst 33342 is bound to the nuclei. Here, each fluorescent marker has a different fluorescence emission wavelength range.

It is particularly noted that, in some embodiments, the first-stage fluorescent staining may further include fluorescently staining the surface antigens of the non-target cells using a fluorescent marker marking the non-target cells. For example, when the target cells are circulating tumor cells, the fluorescent marker in the first stage may further include the anti-CD45 antibody with a specific fluorescence substance, wherein the specific fluorescence substance is, for example, phycoerythrin (PE), which has a fluorescence waveband of excitation wavelength Ex: 554 ± 23 nm/emission wavelength Em: 624 ± 40 nm, which emits orange-yellow fluorescence after being excited. The anti-CD45 antibody may be bound to the surface antigen CD71 of leukocytes (in the present embodiment, leukocytes are the non-target cells), and may be used to mark the position of the non-target cells (e.g., leukocytes). Therefore, in the subsequent step of locating the position of the target cells by fluorescence image analysis, the area emitting orange-yellow fluorescence may be regarded as the area that should be excluded. With this design, the position of the target cells may be more accurately located.

Next, the analysis sample is filled into the cell wells of an array chip by quantitative addition, so that the target cells and the non-target cells in the analysis sample are laid on the bottom of the cell wells in a roughly monolayer manner. In other words, the target cells and the non-target cells are not overlapped in the normal direction of the cell well. The array chip may be, for example, a self-assembly cell array (SACA) chip. The cell self-assembly array chip has multiple groups of hole slots. The bottom of each hole slot is a flat surface having a hydrophilic and anti-cell adhesion coating. Each hole slot includes a cell well and a plurality of evapotranspiration slot. The cell mixed solution is added into the cell well. At this time, the liquid flows from the gap to the evapotranspiration slot, and via the flow field and gravity field driven by the structure, the cells are settled and aligned downward and to the left and right sides. Since the gap is smaller than the cell size, the cells do not flow out of the cell well. As long as the cells in each cell well do not exceed the limit (determined by the volume of the cell well), the cells may be facilitated to be flattened into a single dense layer to effectively improve the image recognition rate of subsequent cells, reduce the probability of misjudgment, and further improve the purity during sorting.

Next, the cell identification system according to an embodiment of the invention is used to identify and locate the target cells contained in the analysis sample in the cell wells of the array chip. The cell identification system provided according to an embodiment of the invention has both a fluorescence image analysis system and a white light image analysis system, and may determine and identify the target cells by an automated image analysis method for the analysis sample in the array chip. The cell identification system of an embodiment of the invention is a cell identification system that may simultaneously achieve automatic cell image location tracking and cell phenotype identification and determination combining fluorescence and white light. The cell identification system interpretation logic program of an embodiment of the invention may utilize the multiple physical and physiological features (including: image fluorescence intensity generated by the interaction of cells with different fluorescent markers, extracellular type classification, and the position of antibody fluorescence on the cells) of the target cells displayed by image recognition for interpretation, and may be combined with artificial intelligence image interpretation logic to perform cell/blood cell identification and deep learning.

The cell identification system provided by an embodiment of the invention at least includes a built-in computer (PC) and a solid-state hard disk (SSD) mounted in the casing for operating the image identification software included in the computer. The cell identification system provided by an embodiment of the invention at least includes a high-magnification microscope equipped with an external LED light source, an XYZ sample moving platform, a dedicated camera, a built-in motor system, an LED light source controller, and an external power supply. The cell identification system provided by an embodiment of the invention at least includes the ability to process eight or more types of sample specimen at a time and take pictures, and each photo may be formed by splicing, for example, 16 small images of individual areas.

First, a fluorescence image of the analysis sample in the cell wells of the array chip is captured using a fluorescence image analysis system, the position of the target cells that emits fluorescence is located, and whether valid fluorescence signals are detected is determined according to the brightness of the fluorescence; according to the degree of overlap of the types of valid fluorescence signals, whether the cells are preliminary target cells is determined. Subsequently, a white light image analysis system is used to capture a white light image of the analysis sample in the cell wells of the array chip. According to the photo of the white light image, the cell phenotype and shape of the preliminary target cells are further interpreted, so as to more accurately identify the target cells. Since the cell identification system according to an embodiment of the disclosure combines fluorescence signal analysis and white light cell phenotype identification at the same time, high accuracy may be achieved even when identifying rare cells in blood.

For example, the array chip provided with the analysis sample is placed on the image analysis machine having the cell identification system according to an embodiment of the invention to perform fluorescence image analysis and white light image analysis. Fluorescence image analysis may be performed, for example, via a fluorescence image analysis system to calibrate the x-y-z axis for the array chip containing the mixed solution on the machine to confirm that the entire array chip is in the correct position, and the relative position of the cells and the cell wells of the array chip may be accurately positioned. White light image analysis is similar to fluorescence image analysis, but white light is used as the light source instead. In the present specification, white light is light with a color temperature in the range of about 3500 K to 10000 K.

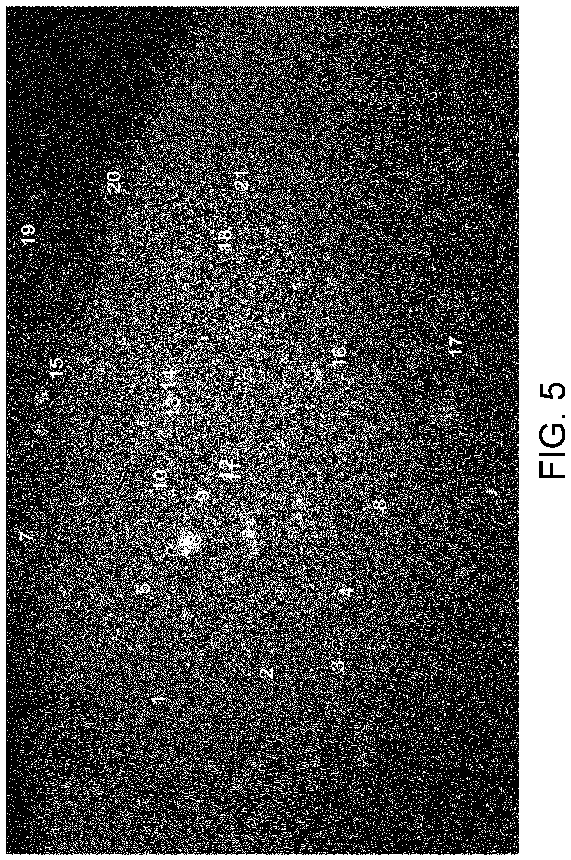

The cell identification system according to an embodiment of the invention first performs fluorescence image analysis. For various fluorescent markers used in the embodiments, fluorescence scan is performed with various specific fluorescent excitation wavebands (the so-called specific fluorescence excitation wavebands mean that the fluorescence excitation wavebands corresponding thereto are used for specific fluorescent markers) and a photo is taken. The photo taken is shown in FIG. 5. For example, fluorescence excitation wavebands of 365 nm (the corresponding fluorescence energy is 6.5 mW), 488 nm (the corresponding fluorescence energy is 13.5 mW), 520 nm (the corresponding fluorescent energy is 15 mW), and 630 nm (the corresponding fluorescence energy is 17.2 mW) are available.

Then, fluorescence intensity analysis is performed on the fluorescence image photo scanned and taken under different fluorescence excitation wavebands. The step of analyzing fluorescence intensity is to interpret the intensity of the fluorescence signal at the position where the fluorescence signal appears according to the captured photo of the fluorescence scan. When the brightness of the fluorescence signal falls within the preset range, the cell identification system according to the invention marks the fluorescence signal as a valid fluorescence signal.

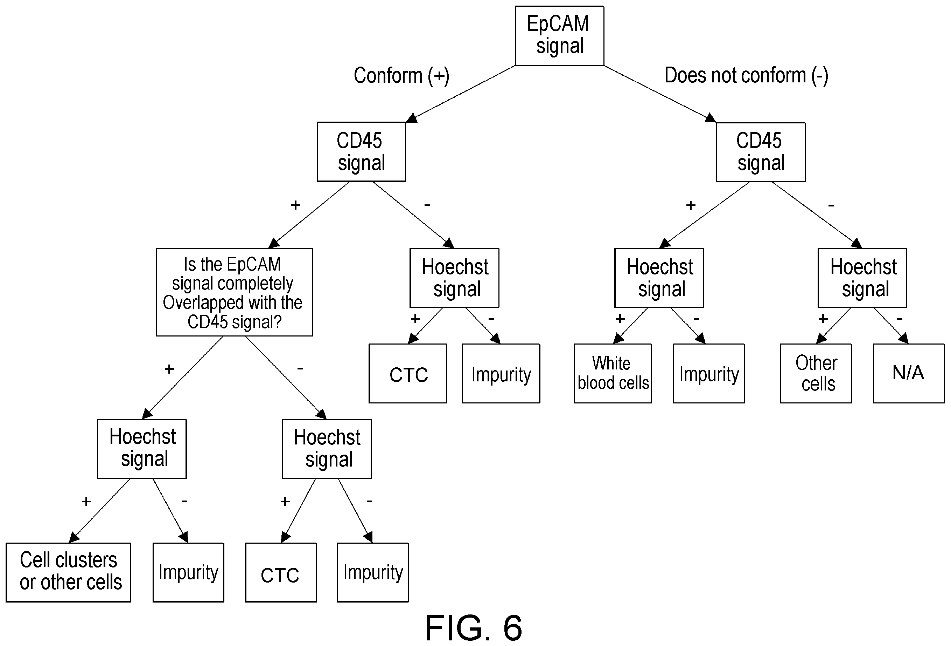

In the first stage, the valid fluorescence signals used to mark the target cells may be interpreted first. For example, when the target cells are circulating tumor cells, the first stage may interpret the fluorescence signal brightness of the anti-EpCAM antibody with FITC to mark the EpCAM signal. Specifically, fluorescence scan is performed using the fluorescence excitation waveband corresponding to FITC (e.g., 488 nm, the corresponding fluorescence energy is 13.5 mW) and a photo is taken, and when the fluorescence signal brightness in the photo falls in the range of 70 to 120, the fluorescence signal is then marked as the valid signal of EpCAM (the EpCAM signal (+) is used as a reference in FIG. 6). According to an embodiment, the cell contour may be drawn by automatic marking or manual circle selection of the valid fluorescence signal area range at this stage.

The second stage may interpret the valid fluorescence signals used to mark non-target cells. For example, the second stage may interpret the fluorescence signal of the anti-CD45 antibody with PE that fluorescently stains non-circulating tumor cells (non-target cells) to exclude non-circulating tumor cells. The anti-CD45 antibody is the surface antigen CD71 bound to white blood cells (i.e., non-target cells) and may be used to mark the location of white blood cells. Specifically, fluorescence scan is performed using the fluorescence excitation waveband corresponding to PE (e.g., 520 nm, the corresponding fluorescence energy is 15 mW) and a photo is taken, and when the fluorescence signal brightness in the photo falls in the range of 3 to 35, the fluorescence signal is then marked as the valid signal of CD45 (the CD45 signal (+) is used as a reference in FIG. 6).

The third stage may interpret the fluorescence substance used to mark specific parts of the cells (for example, the valid fluorescence signal of the fluorescence substance that stains nuclei) to confirm that they are the target cells with the nuclei. For example, the third stage may interpret the fluorescence signal brightness of Hoechst 33342, which fluorescently stains the nuclei, to mark the location where the nucleus signal occurs. Specifically, fluorescence scan is performed using the fluorescence excitation waveband corresponding to Hoechst 33342 (e.g., 365 nm, the corresponding fluorescence energy is 6.5 mW) and a photo is taken, and when the fluorescence signal brightness in the photo falls in the range of 5 to 40, the fluorescence signal is then marked as the valid signal of Hoechst 33342 (the Hoechst signal (+) is used as a reference in FIG. 6).

After marking the various fluorescence signals indicated by the numbers as shown in FIG. 5, fluorescence image analysis may be performed to identify circulating tumor cells according to the interpretation logic flow of FIG. 6. Images captured in different fluorescence wavebands may be stacked for easy comparison. Referring to FIG. 6, if the valid signal of CD45 is also marked at the position where the valid signal of EpCAM is marked, since the valid signal of CD45 is usually indicative of white blood cells rather than circulating tumor cells, it is necessary to further compare whether the valid signal of EpCAM and the valid signal of CD45 are completely overlapped. If the overlap is incomplete and there is a valid signal of Hoechst 33342 at this position, it is preliminarily determined that the cells appearing at this position are circulating tumor cells. If the overlap is complete and there is a valid signal of Hoechst 33342 at this position, the fluorescence brightness of CD45 and the fluorescence brightness of EpCAM need to be further compared. If the fluorescence brightness of CD45 is greater than the fluorescence brightness of EpCAM, the cells appearing at this position are determined to be white blood cells, otherwise, they are circulating tumor cells or circulating tumor cell clusters. Alternatively, if no valid signal of EpCAM is observed at the position where the valid signal of CD45 appears, and there is a valid signal of Hoechst 33342 at this position, it is preliminarily determined that the cells appearing at this position are white blood cells.

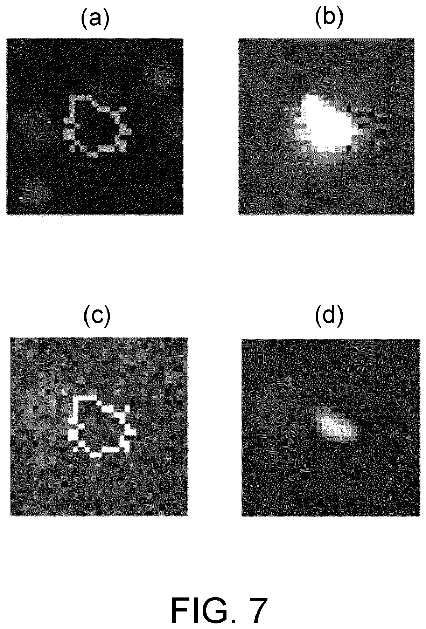

FIG. 7 is an enlarged view of a specific position of a fluorescence image photo of different fluorescence wavebands according to an embodiment of the invention. Referring to FIG. 7, (a) of FIG. 7 is a photo taken by fluorescence scan using the fluorescence excitation waveband corresponding to Hoechst 33342. In the photo, the fluorescence brightness of Hoechst 33342 is 0. (b) of FIG. 7 is a photo taken by fluorescence scan using the fluorescence excitation waveband corresponding to the anti-EpCAM antibody with FITC. The fluorescence brightness in the photo is 220. (c) of FIG. 7 is a photo taken by fluorescence scan using the fluorescence excitation waveband corresponding to the anti-CD45 antibody with PE. The fluorescence brightness in the photo is 26, and (d) of FIG. 7 is a stack of (a) to (c). As shown in FIG. 7, there is a valid signal of EpCAM and a valid signal of CD45 at this position, but there is no valid signal of Hoechst 33342. Therefore, this position should be an impurity.

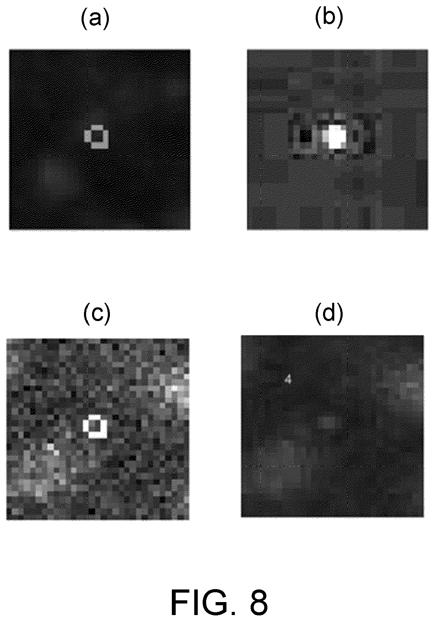

FIG. 8 is an enlarged view of a specific position of a fluorescence image photo of different fluorescence wavebands according to an embodiment of the invention. Referring to FIG. 8, (a) of FIG. 8 is a photo taken by fluorescence scan using the fluorescence excitation waveband corresponding to Hoechst 33342. In the photo, the fluorescence brightness is 22. (b) of FIG. 8 is a photo taken by fluorescence scan using the fluorescence excitation waveband corresponding to the anti-EpCAM antibody with FITC. The fluorescence brightness in the photo is 237. (c) of FIG. 8 is a photo taken by fluorescence scan using the fluorescence excitation waveband corresponding to the anti-CD45 antibody with PE. The fluorescence brightness in the photo is 26, and (d) of FIG. 8 is a stack of (a) to (c). As shown in FIG. 8, the valid signal of EpCAM, the valid signal of CD45, and the valid signal of Hoechst 33342 appear at this position at the same time, but the signal of EpCAM is not completely overlapped with the signal of CD45. Therefore, the cells appearing in this position could be preliminarily determined as target cells.

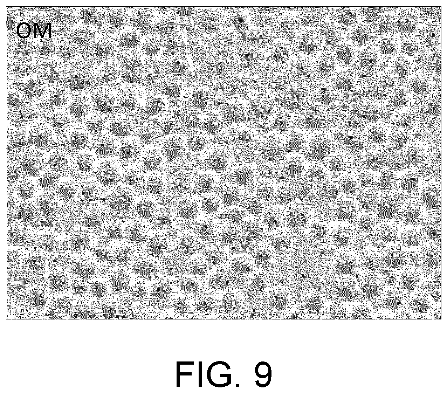

After the fluorescence image analysis is completed, the white light image analysis system is used to calibrate the x-y-z axis of the array chip containing the analysis sample on the machine to confirm that the entire array chip is in the correct position, and the relative positions of the cells and the cell wells of the array chip may be accurately positioned, and white light scan is performed on the analysis sample and a photo is taken. The white light image photo is shown in FIG. 9. Specifically, the cell phenotype and shape of the previously screened preliminary target cells are determined according to the white light image photo. Specifically, the area size of the preliminary target cells and whether the cells appear circular and/or micro-elliptical are interpreted by using the calculation method of perimeter and area. According to the white light image photo, the area size and circumference of the selected preliminary target cells may be calculated, and the white light image photo may be cross-compared with the fluorescence image with the EpCAM fluorescence signal in the above process to confirm whether the relative position error of the cell contour displayed in the white light image photo and the cell contour circled according to the fluorescence signal in the fluorescence image photo is less than or equal to a specific ratio of the diameter of the target cells (for example, less than or equal to 5% of the average diameter of the target cells). The eccentricity may be calculated in combination with the contour of the cell circled by the fluorescence signal. For example, the eccentricity range is about 0 to 0.8. Accordingly, preliminary target cells are selected in the shape of circle and micro-ellipse. When the shape is elliptical (eccentricity may be calculated, for example, eccentricity >0.8 is too elliptical) or non-circular, impurity is defined. Further, the white light image photo is cross-compared with the fluorescence image photo with EpCAM fluorescence signal to confirm whether the relative position error of the positioning of the white light image photo and the fluorescence image photo is less than or equal to 5% of the diameter (for example, circulating tumor cells have an average diameter of about 20 *µ*m) size of the target cells. For example, when the preliminary target cells on the white light image photo have the shape of a circle and a micro-ellipse (the eccentricity may be calculated, for example, an eccentricity greater than 0 but less than 0.8 is regarded as a micro-ellipse), and the error between the position thereof and the position of the valid fluorescence signal of EpCAM is less than or equal to 5% of the diameter of circulating tumor cells (for example, the average diameter of circulating tumor cells is about 20 ,um), the preliminary target cells are identified as target cells.

After the target cells are identified, the target cells may be obtained, for example, by quantitative pipetting at a fixed flow rate (e.g., 20 micro liters per minute (ul/min)) using an automated pipette of a computer-programmable electric single-cell extraction and injection system. Thereby, the function of single-cell extraction may be achieved, and background noise and contamination associated with the sorting solution may be reduced at the same time. The extracted target cells may be further used in procedures such as cell culture, cell biochemical detection, genetic engineering, and further applied in different cytology fields.

By combining the results of fluorescence intensity analysis, fluorescence image analysis, and white light image analysis for cell identification, the accuracy of cell identification may be further improved, so that target cells may be identified with high accuracy. In this way, the cell identification method of the present embodiment may have application value in the fields of cell culture and separation, such as single-cell genomics and proteomics, cell heterogeneity, and the like.

## Claims

1. A cell identification method, comprising:
providing an analysis sample, wherein the analysis sample contains target cells, non-target cells, and fluorescent markers, and the fluorescent markers comprise a first fluorescent marker for marking nuclei, a second fluorescent marker for marking the target cells, and a third fluorescent marker for marking the target cells or the non-target cells;
performing a fluorescence scan on the analysis sample respectively with a first fluorescence waveband corresponding to the first fluorescent marker, a second fluorescence waveband corresponding to the second fluorescent marker, and a third fluorescence waveband corresponding to the third fluorescent marker to obtain a first fluorescence image photo, a second fluorescence image photo, and a third fluorescence image photo of the analysis sample in the first fluorescence waveband, the second fluorescence waveband, and the third fluorescence waveband;
measuring a fluorescence signal brightness of the first fluorescence image photo, the second fluorescence image photo, and the third fluorescence image photo, and marking a fluorescence signal with a fluorescence signal brightness falling within a preset range as a valid fluorescence signal;
marking cells having valid fluorescence signals in both the first fluorescence image photo and the second fluorescence image photo as first preliminary target cells;
marking, when the third fluorescent marker is used to mark the target cells, the first preliminary target cells having valid fluorescence signals in the third fluorescence image photo as second preliminary target cells, or, marking, when the third fluorescent marker is used to mark the non-target cells, the first preliminary target cells that do not have valid fluorescence signals in the third fluorescence image photo as the second preliminary target cells; and
performing a white light scan on the analysis sample with a white light to obtain a white light image photo of the analysis sample, and identifying and screening the second preliminary target cells with a specific cell phenotype and shape as the target cells.

2. The cell identification method of claim 1, wherein the target cells are circulating tumor cells, the first fluorescent marker is Hoechst 33342, the second fluorescent marker is an anti-EpCAM antibody with fluorescein isothiocyanate (FITC), and the third fluorescent marker is an anti-CD45 antibody with phycoerythrin (PE).

3. The cell identification method of claim 1, wherein the target cells are fetal nucleated red blood cells, the first fluorescent marker is 4',6-diamidino-2-phenylindole (DAPI), the second fluorescent marker is an anti-CD147 antibody with FITC, and the third fluorescent marker is an anti-CD71 antibody with PE.

4. The cell identification method of claim 1, wherein the target cells are cells undergoing epithelial-mesenchymal transition, the first fluorescent marker is Hoechst 33342 or DAPI, the second fluorescent marker is an anti-EpCAM antibody with FITC, and the third fluorescent marker is an anti-vimentin antibody with PE.

5. The cell identification method of claim 1, wherein the first fluorescent marker is Hoechst 33342, and a brightness range of a valid fluorescence signal of the first fluorescent marker is 5 to 40.

6. The cell identification method of claim 1, wherein the second fluorescent marker is an anti-EpCAM antibody with FITC, and a brightness range of a valid fluorescence signal of the second fluorescent marker is 70 to 120.

7. The cell identification method of claim 1, wherein the third fluorescent marker is an anti-CD45 antibody with PE, and a brightness range of a valid fluorescence signal of the third fluorescent marker is 3 to 45.

8. The cell identification method of claim 1, wherein the step of performing the white light scan on the analysis sample to obtain the white light image photo of the analysis sample and identifying the second preliminary target cells with the specific cell phenotype and shape as the target cells comprises: calculating, according to the white light image photo of the analysis sample, a perimeter and an area of the marked second preliminary target cells to determine a shape and a size of the marked second preliminary target cells, and if the shape and the size conform to a standard shape of the target cells, the marked second preliminary target cells are determined and identified as the target cells.

9. The cell identification method of claim 8, wherein the standard shape of the target cells comprises a circle or a micro-ellipse with an eccentricity less than 0.8.

10. The cell identification method of claim 8, wherein the step of identifying the second preliminary target cells with the specific cell phenotype and shape as the target cells further comprises: cross-comparing the obtained white light image photo with the second fluorescence image photo, comparing a cell contour of the second preliminary target cells marked in the white light image photo with a cell contour located by the valid fluorescence signals in the second fluorescence image, and if a relative position error of the two contours is less than or equal to a specific ratio of a diameter of the target cells, the second preliminary target cells are determined and identified as the target cells.

11. The cell identification method of claim 10, wherein the specific ratio is 5% of the diameter of the target cells.
